# EUROPÄISCHE PATENTANMELDUNG

(11) **EP 3 085 417 A1**
(43) Veröffentlichungstag der Anmeldung: **26.10.2016**
(21) Anmeldenummer: 15165082.7
(22) Anmeldetag: 24.04.2015
(51) Int. Cl.: A61Q 19/00, A61K 8/34, A61K 8/39, A61K 8/86, A61K 9/00

(54) **GLEITMITTEL**

(71) Anmelder: COR S.a.r.l., 2530 Luxembourg (LU)
(72) Erfinder: Widulle, Herbert, 22547 Hamburg (DE)
(74) Vertreter: Claessen, Rolf

(57) **Zusammenfassung**

Die Erfindung betrifft ein wässriges Gleitmittel für die Anwendung auf der menschlichen Haut oder Schleimhaut enthaltend wenigstens ein Oligopropylenglycol.

## Beschreibung

Die Erfindung betrifft ein wässriges Gleitmittel für die Anwendung auf der menschlichen Haut oder Schleimhaut beim Sexualverkehr. Die Erfindung kann ebenfalls genutzt werden für die Erleichterung des Unwohlseins und der Schmerzen bei vaginaler Trockenheit oder auch für die Behandlung der vaginalen Trockenheit.

Gleitmittel zur Anwendung auf der Haut oder Schleimhaut werden auch während des Sexualverkehrs eingesetzt. Bislang werden für diese Gleitmittel Stoffe wie Glycerin oder Monopropylenglycol eingesetzt. Diese Stoffe erhöhen den Wasserdampfverlust durch die Haut oder Schleimhaut und fördern so das Austrocknen. Bei der Behandlung der vaginalen Trockenheit sind deshalb Produkte, die Glycerin oder Monopropylenglycol enthalten, nicht der Heilung förderlich oder sogar abträglich. Glycerin kann zudem zu Verklebungen führen.

Für Gleitmittel, die beim Sexualverkehr eingesetzt werden, gelten strenge Regelungen. So dürfen diese Gleitmittel beispielsweise keine Peptide bzw. Eiweiße oder biozide Stoffe enthalten. Gleitmittel dürfen nicht zytotoxisch in Sinne der DIN ISO 10 993 - 5 sein. Dies gilt besonders für Gleitmittel, die zur Bekämpfung der vaginalen Trockenheit eingesetzt werden, da diese bei bestimmungsgemäßem Gebrauch über 60 Minuten im Körper verbleiben.

AU 2012203584 B2 beschreibt ein Sonnenschutzmittel mit Eiweißen und anorganischen Partikeln, die potentiell zu Reibung auf der Haut führen können.

EP 2 545 903 A1 beschreibt kosmetische Zusammensetzungen.

WO 2014/074812 A1 beschreibt ein Haarfärbemittel.

Die Aufgabe der vorliegenden Erfindung besteht darin, ein verbessertes Gleitmittel für den Sexualverkehr, für die Bekämpfung von Symptomen und der Behandlung der vaginalen Trockenheit bereitzustellen.

Die der Erfindung zu Grunde liegende Aufgabe wird in einer ersten Ausführungsform durch ein wässriges Gleitmittel für die Anwendung auf der menschlichen Haut oder Schleimhaut gelöst, das dadurch gekennzeichnet ist, dass es wenigstens ein Oligopropylenglycol in einem Bereich von 0,2 bis 70 Gew.% enthält.

Vorzugsweise ist das Gleitmittel für die Anwendung auf der menschlichen Haut oder Schleimhaut beim Sexualverkehr oder zur Anwendung auf der Vaginalschleimhaut ad libitum.

Oligopropylenglycole fördern die Autokonservierung des Gleitmittels, haben keine tensidische Wirkung, entfetten die Haut nicht, penetrieren nicht durch die Schleimhaut und sind ungiftig. Die Oligopropylenglycole sind auch leicht fettend, so dass sie einen entscheidenden weiteren Vorteil haben.

Das erfindungsgemäße Gleitmittel ist vorzugsweise einphasig, ganz besonders bevorzugt keine Emulsion und/oder kein Schaum.

Das erfindungsgemäße Gleitmittel ist vorzugsweise keimarm, pyrogenarm, keimfrei oder pyrogenfrei.

### Propylenglycol

Oligopropylenglycol im Sinne der Erfindung ist vorzugsweise ein Propylenglycol mit 2 bis 12 Propylenglycoleinheiten. Oligopropylenglycol ist besonders bevorzugt ein Dipropylenglycol, Tripropylenglycol oder Tetrapropylenglycol oder eine Mischung dieser Propylenglycole.

Vorzugsweise enthält das Gleitmittel wenigstens ein Oligopropylenglycol in einem Bereich von 0,5 bis 50 Gew.%, insbesondere 2 bis 25 Gew.%.

Das erfindungsgemäße Gleitmittel kann vorzugsweise auch Monopropylenglycol enthalten. Besonders bevorzugt ist Monopropylenglycol in einem Bereich von 1 bis 50 Gew.%, ganz besonders bevorzugt in einem Bereich von 2 bis 25 Gew.% enthalten.

Das Gewichtsverhältnis von Oligopropylenglycol zu Monopropylenglycol liegt vorzugsweise in einem Bereich von 1:5 bis 5:1, ganz besonders bevorzugt 1,1:1 bis 5:1.

### Verdickungsmittel

Vorzugsweise ist in dem Gleitmittel auch wenigstens ein Verdickungsmittel enthalten. Insgesamt kann in dem Gleitmittel vorzugsweise Verdickungsmittel in einem Bereich von 0,1 bis 5 Gew.% enthalten sein.

Verdickungsmittel im Sinne der Erfindung kann auch eine Mischung aus verschiedenen Verdickungsmitteln sein.

So kann wenigstens ein Verdickungsmittel vorzugsweise wasserlöslich sein.

Auch kann wenigstens ein Verdickungsmittel vorzugsweise cellulosebasiert sein. Ganz besonders bevorzugt kann es sich um Hydroxyethylcellulose, Hydropxypropylcellulose und/oder Hydroxymethylcellulose handeln. Das cellulosebasierte Verdickungsmittel kann vorzugsweise in einem Bereich von 0,05 bis 2 Gew.% in dem Gleitmittel enthalten sein.

Auch kann wenigstens ein Verdickungsmittel vorzugsweise Guarkernmehl und/oder Johannisbrotmehl und/oder eine chemische modifizierte Form dieser Verdickungsmittel sein. Das Guarkernmehl und/oder Johannisbrotmehl und/oder die chemisch modifizierte Form dieser Verdickungsmittel kann vorzugsweise in einem Bereich von 0,05 bis 2 Gew.% in dem Gleitmittel enthalten sein.

Auch kann wenigstens ein Verdickungsmittel vorzugsweise Xanthan Gum und/oder algenbasierte Verdickungsmittel und/oder tangbasierte Verdickungsmittel sein. Das Xanthan Gum und/oder algenbasierte Verdickungsmittel und/oder tangbasierte Verdickungsmittel kann vorzugsweise in einem Bereich von 0,05 bis 2 Gew.% in dem Gleitmittel enthalten sein.

Das Gewichtsverhältnis von cellulosebasiertem Verdickungsmittel einerseits zu Guarkernmehl und/oder Johannisbrotmehl und/oder einer chemisch modifizierten Form dieser Verdickungsmittel andererseits kann vorzugsweise in einem Bereich von 0,3:1 bis 1:1 liegen.

### Weitere Bestandteile

Der Wassergehalt im erfindungsgemäßen Gleitmittel kann vorzugsweise wenigstens 50 Gew.%, insbesondere bevorzugt wenigstens 70 Gew.%, ganz besonders bevorzugt wenigstens 80 Gew.% betragen. Unabhängig davon kann der Wassergehalt vorzugsweise bei bis zu 95 Gew.% liegen.

Das erfindungsgemäße Gleitmittel kann vorzugsweise 0,01 bis 0,2 Gew.% Geschmacksstoff oder Süßstoff enthalten.

Das erfindungsgemäße Gleitmittel kann vorzugsweise 0,01 bis 0,5 Gew.% Konservierungsmittel enthalten. Besonders bevorzugt sind Gleitmittel, die kein Konservierungsmittel enthalten und selbstkonservierend sind.

Das erfindungsgemäße Gleitmittel kann vorzugsweise 0,001 bis 0,1 Gew.% Säuren oder deren Salze oder Hydrate, ganz besonders bevorzugt organische Säuren oder deren Salze oder Hydrate enthalten.

### Unerwünschte Bestandteile

Das erfindungsgemäße Gleitmittel enthält vorzugsweise höchstens 0,1 Gew.%, ganz besonders bevorzugt kein Öl oder Fett. Dadurch könnte eine Emulsion entstehen und das Gleitmittel könnte damit mehrphasig werden.

Das erfindungsgemäße Gleitmittel enthält vorzugsweise höchstens 0,1 Gew.%, ganz besonders bevorzugt keine Emulgatoren.

Das erfindungsgemäße Gleitmittel enthält vorzugsweise höchstens 0,1 Gew.%, ganz besonders bevorzugt keine Peptide oder Eiweiße. Diese sind nicht für die Anwendung in der Vagina geeignet, da Peptide oder Eiweiße zu allergischen Reaktionen führen können.

Das erfindungsgemäße Gleitmittel enthält vorzugsweise höchstens 0,1 Gew.%, ganz besonders bevorzugt kein Glycerin. Glycerin kann die Austrocknung der Haut oder Schleimhaut begünstigen.

Das erfindungsgemäße Gleitmittel enthält vorzugsweise höchstens 0,1 Gew.%, ganz besonders bevorzugt keine anorganischen Bestandteile außer Wasser. Diese anorganischen Bestandteile können beispielsweise Silicon oder anorganische Partikel wie keramische Partikel oder Glaspartikel sein. Diese würden zur erhöhten Reibung auf der Haut oder Schleimhaut oder sogar zu Wunden beim Sexualverkehr führen.

Das erfindungsgemäße Gleitmittel enthält vorzugsweise höchstens 0,1 Gew.%, ganz besonders bevorzugt keine vernetzen Polymere.

Das erfindungsgemäße Gleitmittel enthält vorzugsweise höchstens 0,1 Gew.%, ganz besonders bevorzugt keine Parfümöle. Diese sind nicht für die Anwendung in der Vagina zugelassen.

Das erfindungsgemäße Gleitmittel enthält vorzugsweise höchstens 0,1 Gew.%, ganz besonders bevorzugt keine Biozide. Diese sind nicht für die Anwendung in der Vagina zugelassen.

### Ausführungsbeispiele

Drei Zusammensetzungen zu je 1000 g des erfindungsgemäßen Gleitmittels wurden hergestellt, indem die folgenden Bestandteile sorgfältig miteinander verrührt wurden:

| **Bestandteile Beispiel 1** | Gramm |
|---|---|
| Hydroxyethyl Cellulose (CELLOSIZE™ QP 30000H von Dow Chemical Company) | 3 g |
| Guarkernmehl | 7 g |
| Calciumsaccharin | 0,9 g |
| Zitronensäuremonohydrat | 0,2 g |
| Tripropylenglycol | 45 g |
| Monopropylenglycol | 350 g |
| Wasser (auf insgesamt 1000 g aufgefüllt) | ad 1000 g |

| **Bestandteile Beispiel 2** | Gramm |
|---|---|
| Hydroxyethyl Cellulose (CELLOSIZE™ QP 30000H von Dow Chemical Company) | 3 g |
| Guarkernmehl | 7 g |
| Calciumsaccharin | 0,9 g |
| Zitronensäuremonohydrat | 0,2 g |
| Dipropylenglycol | 450 g |
| Wasser (auf insgesamt 1000 g aufgefüllt) | ad 1000 g |

| **Bestandteile Beispiel 3** | Gramm |
|---|---|
| Hydroxypropyl Cellulose (Klucel® MP) | 3 g |
| Guarkernmehl | 7 g |
| Calciumsaccharin | 0,9 g |
| Zitronensäuremonohydrat | 0,2 g |
| Dipropylenglycol | 350 g |
| Tripropylenglycol | 50 g |
| Monopropylenglycol | 25 g |
| Wasser (auf insgesamt 1000 g aufgefüllt) | ad 1000 g |

Die in der vorliegenden Beschreibung sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Wässriges Gleitmittel für die Anwendung auf der menschlichen Haut oder Schleimhaut, **dadurch gekennzeichnet, dass** es wenigstens ein Oligopropylenglycol in einem Bereich von 0,2 bis 70 Gew.% enthält.

2. Gleitmittel gemäß Anspruch 1, **dadurch gekennzeichnet, dass** das wenigstens eine Oligopropylenglycol ein Propylenglycol mit 2 bis 12 Propylenglycoleinheiten ist.

3. Gleitmittel gemäß Anspruch 1 oder 2, **dadurch gekennzeichnet, dass** das wenigstens eine Oligopropylenglycol in einem Bereich von 0,5 bis 50 Gew.%, ganz besonders bevorzugt in einem Bereich von 2 bis 25 Gew.% enthalten ist.

4. Gleitmittel gemäß einem der Ansprüche 1 bis 3, **dadurch gekennzeichnet, dass** das Gleitmittel auch Monopropylenglycol, insbesondere in einem Bereich von 1 bis 50 Gew.%, enthält.

5. Gleitmittel gemäß einem der Ansprüche 1 bis 4, **dadurch gekennzeichnet, dass** Monopropylenglycol in einem Bereich von 2 bis 25 Gew.% enthalten ist.

6. Gleitmittel gemäß einem der Ansprüche 2 bis 5, **dadurch gekennzeichnet, dass** das Gewichtsverhältnis von Oligopropylenglycol zu Monopropylenglycol in einem Bereich von 1,1:1 bis 5:1 liegt.

7. Gleitmittel gemäß einem der Ansprüche 1 bis 6, **dadurch gekennzeichnet, dass** das Gleitmittel auch Verdickungsmittel, insbesondere in einem Bereich von 0,1 bis 5 Gew.%, enthält.

8. Gleitmittel gemäß einem der Ansprüche 1 bis 7, **dadurch gekennzeichnet, dass** das Gleitmittel höchstens 0,1 Gew.%, insbesondere kein Öl oder Fett enthält.

9. Gleitmittel gemäß einem der Ansprüche 1 bis 8, **dadurch gekennzeichnet, dass** das Gleitmittel höchstens 0,1 Gew.%, insbesondere keine anorganischen Bestandteile außer Wasser enthält.

10. Gleitmittel gemäß einem der Ansprüche 1 bis 9, **dadurch gekennzeichnet, dass** das Gleitmittel höchstens 0,1 Gew.%, insbesondere kein Glycerin enthält.
